## Europäisches Patentamt
## European Patent Office
## Office européen des brevets

(19)

(11) Veröffentlichungsnummer: **0 098 512**
**B1**

(12) # EUROPÄISCHE PATENTSCHRIFT

(45) Veröffentlichungstag der Patentschrift:
02.11.88

(51) Int. Cl.⁴: **A 61 F 13/16**

(21) Anmeldenummer: **83106314.4**

(22) Anmeldetag: **29.06.83**

(54) Vorrichtung als Genitalschutz und Monatsbinde.

(30) Priorität: **01.07.82 SE 8204083**

(43) Veröffentlichungstag der Anmeldung:
**18.01.84 Patentblatt 84/3**

(45) Bekanntmachung des Hinweises auf die Patenterteilung:
**02.11.88 Patentblatt 88/44**

(84) Benannte Vertragsstaaten:
**AT BE CH DE FR GB IT LI NL**

(56) Entgegenhaltungen:
**FR-A-2 425 205**
**GB-A-1 580 550**
**US-A-3 417 751**
**US-A-3 452 753**
**US-A-3 658 064**
**US-A-3 860 003**
**US-A-4 050 462**
**US-A-4 337 771**

(73) Patentinhaber: **Landstingens Inköpscentral LIC ekonomisk förening, Svetsarvägen 20, S-171 83 Solna (SE)**

(72) Erfinder: **Beckeström, Bo, Ing., Neptunistigen 40, S-162 40 Vällingby/Schweden (SE)**

(74) Vertreter: **Patentanwälte Dr. Ing. Eugen Maier Dr. Ing. Eckhard Wolf, Pischekstrasse 19, D-7000 Stuttgart 1 (DE)**

LIBER, STOCKHOLM 1988

EP 0 098 512 B1

## Beschreibung

Die Erfindung betrifft eine als Genitalschutz und Monatsbinde dienende Binde der im ersten Teil des Anspruchs 1 angegebenen Gattung.

Es ist eine Binde dieser Art bekannt (US-A-3 452 753), die eine flüssigkeitsdichte, auf der Innenseite in ihrem Mittelfeld eine längliche Absorberschicht tragende Schicht aufweist, deren beiderseits des Mittelfelds an den Längsseiten angeordnete Randstreifen nach einwärts geklappt sind, die Absorberschicht teilweise überlappen und nur an ihren Enden mit den Rändern des Mittelfelds verbunden sind, und an deren einander zugekehrten freien Längsrändern jeweils in einem durch Faltung gebildeten Schlauch ein an seinen Enden mit den Rändern des Mittelfelds verbundenes elastisches Band oder ein elastischer Strang angeordnet ist. Die bekannte Binde weist eine auswechselbare Absorberschicht auf, die über ihre gesamte Länge gleich breit ist und daher auch im mittleren Bereich von den nach einwärts geklappten Randstreifen der äußeren Schicht übergriffen wird. Weiter wird dort als nachteilig empfunden, daß Bänder notwendig sind, um die Binde am Körper zu befestigen.

Weiter ist es bei einem Windelhöschen an sich bekannt (US-A-3 860 003), die Absorberschicht im mittleren Bereich des Windelzuschnitts schmaler als in ihrem Endbereich auszubilden.

Der Erfindung liegt die Aufgabe zugrunde, die bekannte Binde der eingangs angegebenen Art dahingehend zu verbessern, daß sie bei einfacher Ausbildung ohne Zuhilfenahme von zusätzlichen Bändern bequem am Körper anliegt und in der jeweils gewünschten Lage festgehalten wird, so daß auch bei Bewegung der Oberschenkel ein zuverlässiger dichter Abschluß des Schrittbereichs erreicht wird.

Zur Lösung dieser Aufgabe werden die im Anspruch 1 aufgeführten Merkmale vorgeschlagen. Weitere vorteilhafte Ausgestaltungen und Weiterbildungen der Erfindung ergeben sich aus den abhängigen Ansprüchen.

Die äußere flüssigkeitsdichte Schicht des Erfindungsgegenstands weist verhältnismäßig breite, einwärts geklappte, sich über die ganze länge der Binde erstreckende Randstreifen auf, die an ihren Enden mit dem Mittelfeld der äußeren Schicht verbunden sind und an ihren einander gegenüberliegenden freien Rändern ein elastisches Band, einen elastischen Strang oder dergleichen aufweisen. Der gegenseitige Abstand dieser freien Ränder ist gemäß der Erfindung gleich der Breite der Absorberschicht in deren mittleren Bereich. Wenn die Binde im Schritt gegen die Oberschenkel anliegt, legen sich die elastischen Ränder dichtend in Falten der Oberschenkel. Üblicherweise ist diese Lage dadurch gegeben, daß die Binde in einen Slip eingelegt wird. Die Verbindung zwischen den elastischen Rändern und dem mittleren Bereich der Absorberschicht wird gemäß der Erfindung mittels der einwärts geklappten Randstreifen der äußeren Schicht hergestellt, die zusammen wie die Teile eines leicht beweglichen Blasebalgs wirken. Der mittlere Bereich der Absorberschicht kann sich auf diese Weise nach den verschiedensten Richtungen bewegen, da die einwärts geklappten Randstreifen sich wie die seitlichen Falten eines Blasebalgs nach Bedarf mehr oder weniger öffnen, ohne daß diese Bewegungen sich auf die Lage der dichtenden elastischen Ränder auswirken wodurch ein zuverlässiger dichtender Abschluß erzielt wird. Dadurch wird auch das Tragen der Binde wesentlich angenehmer.

In der Zeichnung sind Ausführungsbeispiele der erfindungsgemäßen Binde in schematischer Weise dargestellt. Es zeigen:

Fig. 1 eine Draufsicht auf eine Binde in gestrecktem Zustand unter Spannung der elastischen Ränder;
Fig. 2 eine Seitenansicht der Binde nach Fig. 1;
Fig. 3 einen senkrechten Schnitt nach der Linie 3 - 3 der Fig 1;
Fig. 4 eine Längsseitenansicht der Binde in entspanntem Zustand;
Fig. 5 eine Draufsicht auf die in Fig. 4 dargestellte Binde;
Fig. 6 einen Schnitt nach der Schnittlinie 6 - 6 der Fig. 5;
Fig. 7 bis 9 der Fig. 3 entsprechende Schnitte weiterer Ausführungsbeispiele.

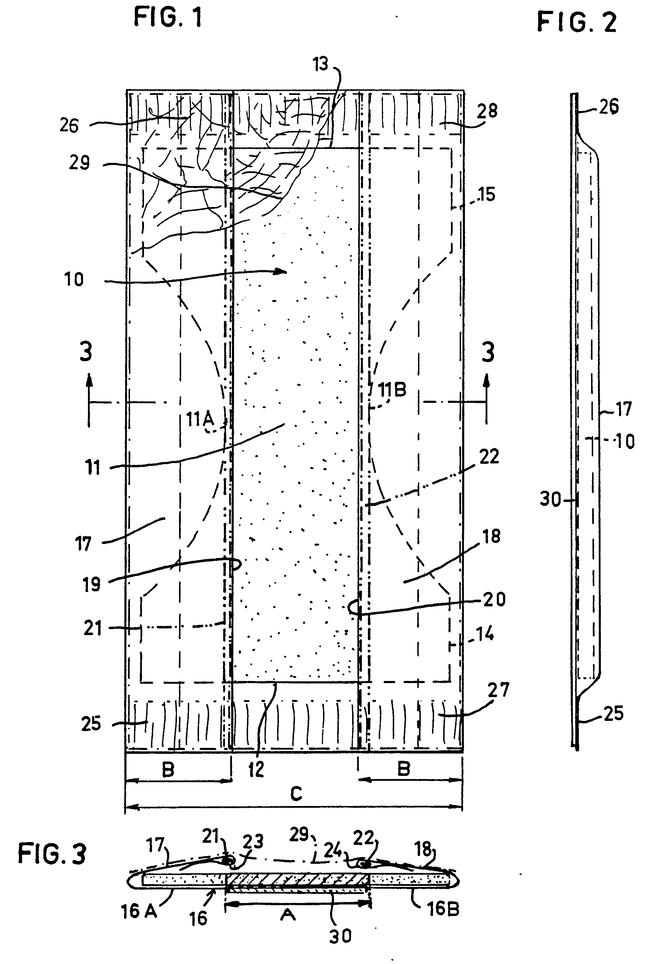

Die Absorberschicht 10 besteht aus einem bekannten flüssigkeitsaufsaugenden Stoff, der in dem in Fig. 1 dargestellten Beispiel in seinem mittleren, zwischen den Enden 12 und 13 gelegenen Bereich 11 eine Breite A aufweist. Diese Breite kann dem Schritt einer Person angepaßt sein, je nachdem die Rinde für Kinder oder Erwachsene bestimmt ist. Bei dem in Fig. 1 dargestellten Beispiel nimmt die Breite der Absorberschicht 10 nach den Enden 14 und 15 zu und beträgt an den Enden etwa das Doppelte der Breite im mittleren Bereich 11.

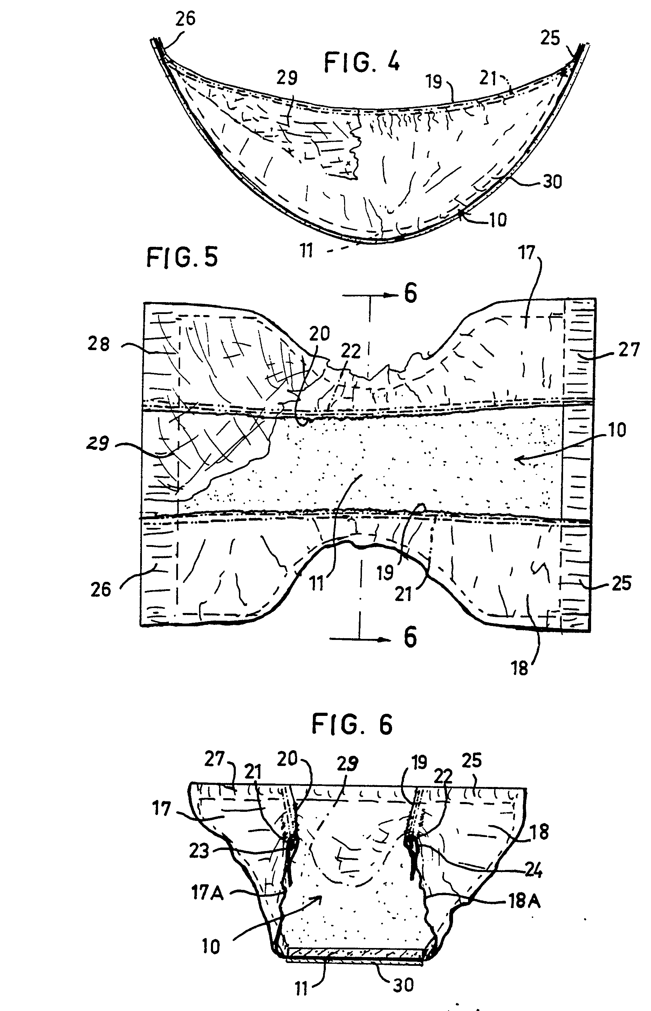

Die Absorberschicht 10 ist mit einer äußeren flüssigkeitsdichten Schicht 16 verbunden, die durch eine Kunststoff-Folie gebildet sein kann und die sowohl in ihrer Länge als auch in ihrer Breite größere Abmessungen als die Absorberschicht 10 aufweist. Die verhältnismäßig breiten Randstreifen 17, 18 der Schicht 16 sind nach innen geklappt und weisen an ihren freien Rändern 19, 20 ein elastisches Band 21, 22 auf, das in Fig. 1 gespannt ist, während diese elastischen Bänder in der Darstellung der Fig. 4 bis 6 sich zusammengezogen haben, so daß die freien Ränder 19, 20 eine Faltenbildung aufweisen.

Die Ränder der Randstreifen 17, 18 sind umgefaltet und als Schläuche 23, 24 ausgebildet, die in sich die elastischen Bänder 21, 22 aufnehmen, die in gespanntem Zustand, wie aus Fig. 1 ersichtlich, an ihren Enden mit den Enden 25, 26 bzw. 27, 28 der Randstreifen 17, 18 verbunden sind, die ihrerseits mit den Endbereichen des Mittelfeldes der äußeren Schicht 16 verbunden sind, wie dies in Fig. 1 durch dünne, nicht zusammenhängende Striche angedeutet ist.

Auf die einwärts geklappten Randstreifen 17, 18 ist eine Deckschicht 29 aufgebracht, von der in die Fig. 1, 4 und 5 nur ein Randbereich eingezeichnet ist. Diese Deckschicht besteht aus flüssigkeitsdurchlässigem, gewebtem oder gewirktem textilen Stoff und ist mit den Randstreifen 17, 18 und den Endbereichen des Mittelfeldes der äußeren Schicht 16 verbunden. Auf dieses Mittelfeld ist, wie aus Fig. 3 ersichtlich, ein Streifen 30 mit einer die Haftwirkung erhöhenden rauhen Oberfläche aufgebracht, die nach dem Einlegen in einen Slip oder dergleichen an der Innenseite des Slips haftet und so die Binde in der jeweils gewünschten Lage festhält.

Die Breite B der Randstreifen 17, 18 beträgt ungefähr ein Viertel oder Drittel der Breite C der in gespanntem Zustand befindlichen Binde, wie dies aus Fig. 1 ersichtlich ist. Die Breite der einwärts geklappten Randstreifen 17, 18 kann jedoch auch kleiner oder größer sein und beispielsweise die Hälfte oder nur ein Zehntel der Breite C betragen.

Wie aus Fig. 1 ersichtlich ist, liegen die freien Ränder 19, 20 im gespannten Zustand der Binde etwa an den Seitenrändern 11A, 11B der Absorberschicht 10 in deren mittlerem Bereich 11 an. In diesem Bereich wirken die Randstreifen 17, 18 im Zusammenwirken mit den außerhalb der Randstellen 11A, 11B gelegenen, keine Absorberschicht aufweisenden Teilen 16A, 16B der Schicht 16 wie ein Blasebalg.

Im entspannten Zustand, wie er in den Fig. 4 bis 6 dargestellt ist, öffnen sich die so gebildeten Falten, so daß die Absorberschicht 10 in ihrem mittleren Bereich 11 zwischen den sich aufrichtenden Teilen 17A, 18A der Randstreifen 17, 18 hängt (Fig. 6).

Wenn die Binde an ihren Enden ergriffen und in ihrer Gebrauchslage zwischen den Schenkeln hochgezogen wird, gelangen die elastischen Ränder 19, 20 in abdichtenden und elastischen Kontakt mit den im Schrittbereich vorhandenen Falten der Oberschenkel. Wenn dann der Slip angezogen wird, preßt dieser die Absorberschicht 10 in die Verengung zwischen den Schenkeln. Die Teile 17A, 18A der Randstreifen 17, 18 richten sich hierbei in einem kleineren oder größeren Maße auf und bilden eine bewegliche Verbindung zwischen den elastischen Rändern 19, 20 und dem mittleren Bereich 11 der Absorberschicht 10, so daß dieser ohne Auswirkung auf die elastischen Ränder 19, 20 und deren Abdichtung sich verhältnismäßig frei bewegen kann.

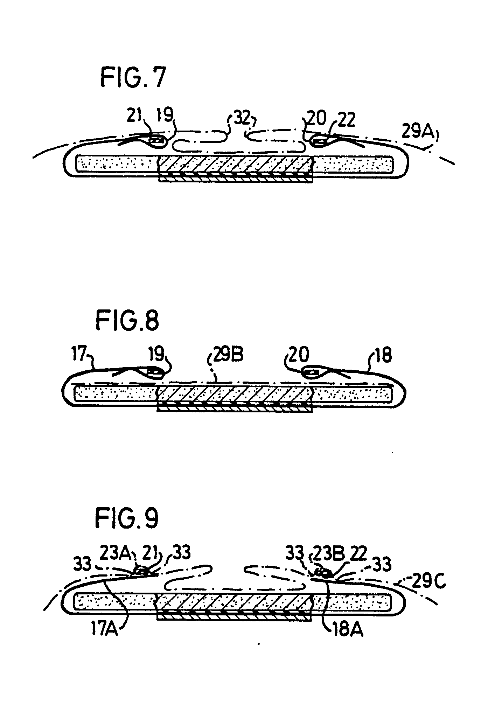

Das Ausführungsbeispiel nach Fig. 7 unterscheidet sich von dem Ausführungsbeispiel der Fig. 1 bis 3 nur dadurch, daß die nicht gewebte Deckschicht 29A zwischen den elastischen Rändern 19, 20 mehrere Falten 32 zur Aufnahme eines männlichen Glieds aufweist.

Das Ausführungsbeispiel nach Fig. 8 weist eine nicht gewebte Deckschicht 29B auf, die gegen die Absorberschicht 10 anliegt und sich bis zu deren Seitenrändern erstreckt, so daß die Randstreifen 17, 18 sich gegenüber der nicht gewebten Deckschicht 29B frei bewegen können und das männliche Glied sich gegen die Absorberschicht 10 anlegen kann.

Bei dem Ausführungsbeispiel nach Fig. 9 sind die elastischen Bänder 21, 22 in Schläuchen 23A, 23B angeordnet, die von der nicht gewebten Deckschicht 29G gebildet werden, die längs der Linien 33 zu beiden Seiten der elastischen Bänder 21, 22 mit den Randstreifen 17A, 18A verschweißt oder verklebt ist.

**Patentansprüche**

1. Als Genitalschutz und Monatsbinde dienende Binde mit einer flüssigkeitsdichten, auf der Innenseite in ihrem Mittelfeld eine längliche Absorberschicht (10) tragenden äußeren Schicht (16), beispielsweise einer Kunststoff-Folie, deren beiderseits des Mittelfeldes an den Längsseiten angeordnete Randstreifen (17, 18) nach einwärts geklappt sind, die Absorberschicht (10) teilweise überlappen und nur an ihren Enden mit den Rändern des Mittelfeldes verbunden sind, und an deren einander zugekehrten freien Längsrändern (23, 24) ein an seinen Enden mit den Rändern des Mittelfeldes verbundenes elastisches Band (21, 22) oder ein elastischer Strang angeordnet ist, dadurch gekennzeichnet, daß die mit der äußeren Schicht (16) verbundene Absorberschicht (10) in ihrem in Längsrichtung gesehen mittleren Bereich (11) eine gegenüber ihren Endbereichen (12, 13) und gegenüber dem Mittelfeld der äußeren Schicht (16) geringere, ungefähr dem Abstand der Längsränder (19, 20) der beiden einwärts geklappten Randstreifen (17, 18) entsprechende Breite (A) aufweist und daß auf der Außenseite des Mittelfeldes der äußeren Schicht (16) ein eine haftende Oberfläche aufweisender Streifen (30) aufgebracht ist.

2. Binde nach Anspruch 1, dadurch gekennzeichnet, daß die beiden einwärts geklappten Randstreifen (17, 18) eine Breite (B) zwischen einem Drittel und einem Viertel der Breite (C) der äußeren Schicht (16) aufweisen.

3. Binde nach den Ansprüchen 1 und 2, dadurch gekennzeichnet, daß sie eine über die beiden einwärts geklappten Randstreifen (17, 18) sich erstreckende Deckschicht (29) aus flüssigkeitsdurchlässigem Stoff aufweist.

4. Binde nach Anspruch 3, dadurch gekennzeichnet, daß die Deckschicht (29A) in ihrem mittleren, zwischen den Längsrändern (19, 20) der Randstreifen (17, 18) der äußeren Schicht (16) gelegenen Bereich zwei Falten (32) aufweist.

5. Binde nach Anspruch 1 oder 4 bei der das Merkmal, daß das elastische Band (21, 22) jeweils in einem durch Faltung gebildeten Schlauch (23, 24) an den freien Längsrändern (19, 20) der Randstreifen (17, 18) angeordnet ist, dadurch ersetzt ist, daß das elastische Band (21, 22) jeweils in einem Schlauch (23A, 23B) angeordnet ist, welcher dadurch gebildet ist, daß eine bzw. die Deckschicht (29C) längs zwei paralleler Linien (33) mit den einwärts geklappten Rand streifen (17, 18 bzw. 17A, 18A) verschweißt oder verklebt ist.

6. Binde nach Anspruch 1 oder 2, dadurch gekennzeichnet, daß sie eine unter die beiden einwärts geklappten Randstreifen (17, 18) sich erstreckende Deckschicht (29B) aus flüssigkeitsdurchlässigem Stoff aufweist. (Fig. 8)

## Claims

1. Diaper used for genital protection and as a sanitary towel, having an outer layer (16) which is impermeable to liquid, for example a plastic sheet, holding an oblong absorbent layer (10) in its centre panel on the inner face, the edge flaps (17, 18) of said first, impermeable layer which are disposed either side of the centre panel on the long sides being folded inwards, partially overlapping the absorbent layer (10) and only joined at their ends to the edges of the centre panel, and on each of the free, facing long edges (23, 24) thereof there being arranged an elasticated band (21, 22) or elasticated cord, which is joined at its ends to the edges of the centre panel, characterised in that the absorbent layer (10) joined to the outer layer (16) has in its central portion (11), viewed lengthways, a lesser width (A) relative to its end portions (12, 13) and relative to the centre panel of the outer layer (16), roughly corresponding to the distance between the long edges (19, 20) of the two inwardly folded edge flaps (17, 18), and that to the outside of the centre panel of the outer layer (16) is applied a strip (30) having an adhesive surface.

2. Diaper according to Claim 1, characterised in that the width (B) of the two inwardly folded edge flaps (17, 18) is between one-third and one-quarter the width (C) of the outer layer (16).

3. Diaper according to Claims 1 and 2, characterised in that it has a top sheet (29) made from a material permeable to liquid and extending over the two inwardly folded edge flaps (17, 18).

4. Diaper according to Claim 3, characterised in that in its central portion, lying between the long edges (19, 20) of the edge flaps (17, 18) of the outer layer (16), the top sheet (29A) has two folds (32).

5. Diaper according to Claim 1 or 4, wherein the feature that the elasticated band (21, 22) is, in each case in a tube (23, 24) formed by folding, arranged on the free long edges (19, 20) of the edge flaps (17, 18), is replaced by arranging the elasticated band (21, 22), in each case in a tube (23A, 23B) formed by bonding or glueing a top sheet (29C), or the top sheet (29C), along two parallel lines (33) to the inwardly folded edge flaps (17,18 and 17A, 18A, respectively).

6. Diaper according to Claim 1 or 2, characterized in that it has a top sheet (29B) made from a material permeable to liquid, which extends beneath the two inwardly folded edge flaps (17, 18) (Fig 8).

## Revendications

1. Bande servant de protection génitale et de serviette hygiénique et comprenant une couche externe imperméable (16), une feuille de matière plastique par exemple, qui porte, à la face intérieure de sa partie médiane, une couche absorbante (10) oblongue et dont les bandes latérales (17, 18), qui sont disposées sur les côtés longitudinaux de part et d'autre de la partie médiane, sont rabattues vers l'intérieur, recouvrent en partie la couche absorbante (10) et ne sont reliées aux bords de la partie médiane que par leurs extrémités, un ruban élastique (21, 22), qui est relié par ses extrémités aux bords de la partie médiane, ou un cordon élastique, étant disposé le long des bords longitudinaux (19, 20) libres, tournés l'un vers l'autre, desdites bandes latérales, caractérisée en ce que la couche absorbante (10), qui est reliée à la couche externe (16), présente, dans sa partie médiane (11) lorsque l'on regarde dans le sens de la longueur, une largeur (A) qui est réduite par rapport aux extrémités (12, 13) de ladite couche et par rapport à la partie médiane de la couche externe (16) et qui correspond sensiblement à la distance entre les bords longitudinaux (19, 20) des deux bandes latérales (17, 18) rabattues vers l'intérieur et en ce qu'un ruban (30), qui comporte une face extérieure adhésive, est placé à la face extérieure de la partie médiane de la couche externe (16).

2. Bande selon la revendication 1, caractérisée en ce que les deux bandes latérales (17, 18) rabattues vers l'intérieur ont une largeur (B) qui se situe entre un tiers et un quart de la largeur (C) de la couche externe (16).

3. Bande selon les revendications 1 et 2, caractérisée en ce qu'elle comporte une couche de recouvrement (29) qui est constituée d'une matière perméable et qui s'étend sur les deux bandes latérales (17, 18) rabattues vers l'intérieur.

4. Bande selon la revendication 3, caractérisée en ce que la couche de recouvrement (29A), dans sa partie médiane qui se situe entre les bords longitudinaux (19, 20) des bandes latérales (17, 18) de la couche externe (16), présente deux plis (32).

5. Bande selon la revendication 1 ou 4, dans laquelle la caractéristique qui consiste en ce que chaque ruban élastique (21, 22) est disposé dans une boucle (23, 24) formée par pliage des bords longitudinaux (19, 20) libres des bandes latérales (17, 13) est modifiée en ce que chaque ruban élastique (21, 22) est placé dans une boucle (23A, 23B) qui est formée en soudant ou collant une ou la couche de recouvrement (29C) aux bandes latérales (17, 18 ou 17A, 18A) rabattues vers l'intérieur, le long de deux lignes parallèles (33).

6. Bande selon la revendication 1 ou 2, caractérisée en ce qu'elle comporte une couche de recouvrement (29B) qui est constituée d'une matière perméable et qui s'étend en dessous des deux bandes latérales (17, 18) rabattues vers l'intérieur. (Figure 8).

FIG. 1

FIG. 2

FIG. 3

FIG. 4

26    29    19    21    25    30    10    11

FIG. 5

6

28    20    22    17    27

29    10

26    11    19    21    25    18

6

FIG. 6

27    21    20    29    19    22    25
17    18
23    24
17A    18A
10    11    30

## FIG. 7

## FIG. 8

## FIG. 9